# EUROPEAN PATENT APPLICATION

(11) **EP 4 113 531 A1**
(43) Date of publication of application: **04.01.2023**
(21) Application number: 21182362.0
(22) Date of filing: 29.06.2021
(51) Int. Cl.: G16H 40/40

(54) **PREDICTIVE MAINTENANCE SYSTEM AND METHOD**

(71) Applicant: Radiometer Medical ApS, 2700 Brønshøj (DK)
(72) Inventor: EIERDAL, Lars, 2700 Brønshøj (DK); DANIELSEN, Martin Tandal Srichan, 2700 Brønshøj (DK); LARSEN, Allan Sandbjerg, 2700 Brønshøj (DK)
(74) Representative: Radiometer IPR

(57) **Abstract**

A computer-implemented method for supporting maintenance of an apparatus for analyzing biological samples, the apparatus including at least one hardware component subject to maintenance, the method comprising: receiving a plurality of measurement results, each measurement result being derived from a measurement performed by the apparatus for analyzing biological samples on a biological sample or on a quality control sample, the plurality of measurement results including measurement results obtained at different times; computing a time series of a performance parameter from the plurality of measurement results, the performance parameter being indicative of an operational state of said at least one hardware component, the time series representing a period of time having a start time and an end time, the end time being no later than a current time; extrapolating, using a predetermined extrapolation model, the computed time series to obtain an extrapolated time series of the performance parameter, the extrapolated time series including one or more estimated values of the performance parameter at one or more future times; comparing the one or more estimated values of the performance parameter with a predetermined condition indicative of a risk of component failure of said component to determine an estimated future time at which said predetermined condition is fulfilled; determining an estimated failure time from the estimated future time.

## Description

### TECHNICAL FIELD

The present invention relates in one aspect to a method and a system for supporting maintenance of an apparatus for analyzing biological samples, such as a body fluid analyzer, in particular a blood analyzer.

### BACKGROUND

Analyzer units for measuring physical parameters of analytes in biological samples, in particular in liquid samples, by means of respective detectors or sensors are widely used in various industries, such as food industry, environmental industry, as well as medical and clinical industry. Such analyzer units are often simply referred to as analyzers.

In addition to general requirements in respect of accuracy, precision, and reliability, analyzer units for clinical applications are often subject to further critical constraints. Such constraints include a need for low operating costs, low down times, ease of use, etc.

The combination of some or even all of these constraints is particularly relevant in point-of-care analyzers, such as blood analyzers. Point-of-care blood analyzers are often used by hospital wards to provide measurements of various parameters for analyzing the blood of human patients treated in the hospital ward, e.g. for establishing and/or monitoring a biological condition of the patient. In a variety of instances it is desirable to measure e.g. the partial pressure of blood gasses in a whole blood sample of the mammal subject, concentrations of electrolytes and/or metabolites in the blood sample, as well as the hematocrit value of the blood sample. For example, measuring *p*CO₂, *p*O₂*,* pH, Na⁺, K⁺, Ca²⁺, Cl-, Mg²⁺, glucose, lactate, creatinine, urea and hemoglobin and hemoglobin-derivate values are primary clinical indications in assessing the condition of a medical patient. Based on such measurements, immediate decisions on any treatment of the patient are frequently taken. A number of different analyzers currently exist for making such measurements of at least some of the above parameters. Such analyzers are able to perform precise measurements in order to provide the most meaningful diagnostic information. The analyzers typically display the measurement results on a display or print the results on a slip of paper shortly after performing the analysis so as to allow the healthcare personnel to make immediate use of the measurement results.

The ability to efficiently and reliably perform a large number of blood analyses in a timely manner and at all times is important for many healthcare facilities. For example, some patients in intensive care may require a sampling frequency of 15-20 samples per day for blood gas and clinical chemistry measurements, leading to a potentially large number of blood samples to be analyzed each day. The availability of the analyzers at all times is not only important for the medical decision making but also in order to reduce operational costs.

Therefore, in the context of blood analyzers as well as in the context of apparatus for analyzing biological samples in general, apparatus maintenance is an important aspect of the operation of such apparatus. It is generally desirable that proper functioning of the apparatus for analyzing biological samples is ensured as much as possible. In particular, it is desirable that unexpected apparatus failures and unplanned maintenance be reduced.

In this respect, so-called corrective maintenance generally aims at addressing existing errors of an apparatus as and when it is detected that such errors have occurred. This paradigm thus often results in unplanned maintenance and/or extended down-time. Preventive maintenance seeks to avoid unexpected errors by performing periodic maintenance without considering the current operational status of an apparatus. Accordingly, preventive maintenance may result in unnecessary maintenance, e.g. including unnecessary replacements of parts.

The conference paper "Predictive Maintenance in Healthcare Services with Big Data Technologies" by S. Gokalp et al., DOI: 10.1109/SOCA.2018.00021, describes a scalable predictive maintenance architecture for the healthcare domain. Predictive maintenance seeks to forecast when maintenance may be required.

US 2014/0266713 discloses a method for generating an alert to perform maintenance on a medical device based on actual usage of the medical device. This prior art method includes measuring a parameter value for a parameter type. The parameter type can be associated with a component in a medical device connected to a network and related to a function performed by the component. The method further includes locally storing at the medical device the measured parameter value, the parameter type, and a first value that indicates when the measured parameter value was measured; accessing a maintenance threshold value that specifies a performance limit for the parameter type; comparing the maintenance threshold value with the measured parameter value; and generating an alert based on the comparing to indicate that maintenance is needed for the component.

However, it remains desirable to provide a method and system that allows an accurate and reliable prediction of component malfunctions of an apparatus for analyzing biological samples.

### SUMMARY

On this background, according to a first aspect, disclosed herein are embodiments of a computer-implemented method for supporting maintenance of an apparatus for analyzing biological samples, the apparatus including at least one hardware component subject to maintenance, the method comprising:
- receiving a plurality of measurement results, each measurement result being derived from a measurement performed by the apparatus for analyzing biological samples on a biological sample or on a quality control sample, the plurality of measurement results including measurement results obtained at different times;
- computing a time series of a performance parameter from the plurality of measurement results, the performance parameter being indicative of an operational state of said at least one hardware component, the time series representing a period of time having a start time and an end time, the end time being no later than a current time;
- extrapolating, using a predetermined extrapolation model, the computed time series to obtain an extrapolated time series of the performance parameter, the extrapolated time series including one or more estimated values of the performance parameter at one or more future times later than the end time, in particular later than the current time;

- comparing the one or more estimated values of the performance parameter with a predetermined condition indicative of a risk of component failure of said component to determine an estimated future time at which said predetermined condition is fulfilled;
- determining an estimated failure time from the estimated future time.

Accordingly, as the determination of the estimated failure time is based on actual measurements of the apparatus, embodiments of the method disclosed herein provide an accurate and reliable prediction of a time at which the hardware component is likely to fail, i.e. to cease operating sufficiently well, to cease operating at all or even to break.

In some embodiments, the plurality of measurement results includes one or more measurement results derived from a measurement performed by the apparatus for analyzing biological samples on a quality control sample. In some embodiments, the plurality of measurement results includes one or more measurement results derived from a measurement performed by the apparatus for analyzing biological samples on a quality control sample and one or more measurement results derived from a measurement performed by the apparatus for analyzing biological samples on a biological sample. Accordingly, the prediction is based on the normal operation of the apparatus as well on well controllable quality control procedures. Basing the computation of the time series of the performance parameter at least in part on measurements on a quality control sample, in particular when the computation is based on measurements on biological samples as well as on measurements on a quality control sample, allows a particularly reliable determination of the estimated failure time. In this context, the term quality control sample refers to a sample, such as a liquid sample, having a known composition and having a known target value of the measurement result, thus allowing an accurate determination of a deviation between the measurement result derived from a measurement performed by the apparatus for analyzing biological samples on the quality control sample and the target value associated with said quality control sample. Typically, quality control samples are synthetically manufactured samples, i.e. samples not obtained from a patient.

Moreover, at least some embodiments of the method do not require additional measurements, which would not have been performed otherwise. In particular, the determination may be based on measurements of biological samples performed as part of the normal operation of the apparatus and/or quality control measurements on quality control samples performed as part of the normal routine quality control of the apparatus.

The determination of the estimated failure time based on an extrapolation of a computed time series is computationally efficient and can be performed based on relatively little data, in particular based on few measurements.

The extrapolation model may be a predetermined model which may be based on a priori knowledge of known causes of component failures and/or known indicators for component failures. The extrapolated time series may be in the form of one or more discrete estimated future values or in the form of a continuous extrapolation function. The extrapolation function may be indicative of a plurality of estimated future values at respective times in the future, later than the current time.

In some embodiments, extrapolating comprises performing a linear extrapolation, i.e. the extrapolation model is a linear model, thus resulting in a computationally efficient and robust method. In other embodiments other types of extrapolations, i.e. other types of extrapolation models may be used, such as non-linear models or time-series prediction models based on machine learning.

The predetermined condition indicative of a risk of component failure may be a suitable condition selected based on prior knowledge about which level or property of the performance parameter indicates a high risk of component failure. In some embodiments, the condition relates to a performance parameter moving outside of a predetermined acceptable range. Accordingly, comparing may comprise comparing the one or more estimated values of the performance parameter with a predetermined target range of the performance parameter to determine an estimated future time at which one or more of the estimated values lie outside the predetermined target range. In other embodiments, other conditions may be used, e.g. comparing another attribute of the estimated future values with an acceptable range for such attribute to determine an estimated future time where such attribute falls outside the acceptable range. An example of attributes includes a rate of change of the performance parameter over time, an amplitude or frequency of a fluctuation of the performance parameter with an acceptable range, etc.

In some embodiments, the method further comprises determining a confidence interval associated with the determined estimated failure time, e.g. a 95% confidence interval or the like.

The determination of the estimated failure time based on an extrapolation of a time series may easily be visualized or otherwise presented to an operator of the apparatus, thus facilitating an informed decision-making process. Generally, the method may comprise outputting the estimated failure time. The estimated failure time may be output via a suitable user-interface, such as a graphical user-interface of a data processing system.

Generally, the performance parameter is indicative of an operational state of the at least one hardware component. In this regard, the operational state of the hardware component includes the degree of deterioration, wear and tear and/or other factors indicative of the component approaching the end of its usable operational life or the component requiring maintenance in order the remain usable. Accordingly, the performance parameter being indicative of an operational state of said at least one hardware component involves the performance parameter being chosen such that there is a correlation between the performance parameter and the operational state of the hardware component that allows a prediction or determination of a deterioration or imminent component failure to be made. It will be appreciated that the choice of performance parameter for the purpose of predicting an estimated failure time of a hardware component may depend on the hardware component in question and may e.g. be based on a priori knowledge about how deterioration of a particular hardware component affects measurement results. In some embodiments, the selection of a suitable performance parameter may be based on a statistical analysis of data obtained during operation of one or more apparatus for analyzing biological samples, e.g. so as to determine a correlation between measurement results or combinations of measurement results and observed component failures.

The performance parameter may be a single measured parameter, obtained by a measurement of a biological sample or a quality control sample, or a combination of multiple measured parameters and/or a combination of multiple measurements of a single parameter. In some embodiments, the performance parameter may further be based on additional operational data associated with the apparatus, such as a power consumption, consumption of one or more substances, reference measurements of light intensities or other parameters associated with a detector, etc. In some embodiments, estimation of a failure time may be performed based on a combination of more than one performance parameters.

In some embodiments, the performance parameter is indicative of a deviation of a measured value from a target value associated with a quality control sample, the measured value resulting from a measurement of a quantity performed by the apparatus on said quality control sample. It has turned out that an extrapolation of such deviations is a reliable indicator of component failure in an apparatus for analyzing biological samples. The measured value may be a value directly obtained from the measurement or obtained from a measurement and from a subsequent preprocessing.

In some embodiments, the performance parameters are at least in part based on one or more measurement results resulting from a measurement of one or more biological samples by the apparatus for analyzing biological samples. As some measurements can only, or at least more accurately, be performed on actual biological samples, this facilitates a more accurate estimate of the predicted failure time.

In some embodiments, the measured value is a corrected measured value resulting from a measurement of a quantity performed by the apparatus on said quality control sample and corrected based on one or more previous measurements performed on respective biological samples.

The start and end time associated with the computed time series define an observation period where the start time lies in the past (relative to a current time at which the computation is performed) while the end time may correspond to the current time or also lie in the past. For example, the end time may be the time of the most recent measurement result. Computing the time series comprises one or more data cleansing operations, such as noise reduction, outlier elimination, filtering, moving average and/or moving median.

In some embodiments, computing the time series comprises:
- detecting one or more operational events, and
- discarding one or more of the received measurement results, the one or more discarded measurement results resulting from measurements performed at a time subsequent to the detected operational event. The one or more discarded measurement results may result from measurements performed during a predetermined period after the detected event. Accordingly, in situations where certain operational events are known or suspected to affect the performance parameter, and efficient mechanism is provided for increasing the reliability of the resulting estimated failure time. Detecting the one or more operational events may comprise detecting one or more predetermined operational events, such as a restart/reboot of the apparatus, the performance of one or more maintenance activities, such as replacement of parts, etc.

In some embodiments, the determination of the estimated failure time is performed by the apparatus for analyzing biological samples itself. To this end, the apparatus may comprise a suitably programmed processing unit. The resulting estimated failure time may be output via a suitable user-interface of the apparatus and/or communicated to a remote data processing system, e.g. a central management system or maintenance support system. Alternatively, the determination of the estimated failure time may completely or in part be performed by a data processing system remote from the apparatus for analyzing biological samples. The remote data processing system may be a suitably programmed server computer, a cloud-based computing architecture, or the like. To this end, the apparatus for analyzing biological samples may be communicatively coupled to the remote data processing system and forward the measurement results. Here and in the following the term measurement results comprises raw, i.e. unprocessed sensor/detector data measured by one or more sensor/detector and/or or pre-processed sensor/detector data and/or measurement results computed from the sensor/detector data and/or the performance parameter and/or other suitable input for the computation of the estimated failure time to the remote data processing system. Accordingly, receiving the measurement results may comprise receiving the measurement results from the apparatus via a communications network and performing some or all of the steps of computing, extrapolating, comparing and determining by a data processing system remote from the apparatus.

It will be appreciated that the apparatus for analyzing biological samples may be communicatively coupled to the remote data processing system via any suitable wired and/or wireless communications interface, e.g. via a suitable communications network, such as a local computer network and/or the internet. The remote data processing system may output the determined estimated failure time via a suitable user-interface and/or communicate the determined estimated failure time to a client device, such as client computer, client tablet, client terminal etc. For example, the client device may be a suitable computing device configured to provide a user-interface, e.g. a web-based user interface for displaying the determined estimated failure time. Alternatively or additionally, the remote data processing system may communicate the determined estimated failure time to the apparatus for analyzing biological samples and/or to another data processing system, such as a maintenance support system, an inventory management system and/or the like.

It will be appreciated that the determined estimated failure time may be output and/or communicated along with other data pertinent to a particular apparatus, e.g. an apparatus serial number or other information identifying the apparatus, other data related to the operational status and/or maintenance of the apparatus, etc.

In some embodiments, the apparatus is a blood analyzer for analyzing blood samples, i.e. the biological samples may be blood samples, in particular samples of whole blood. The blood analyzer may be configured to measure the partial pressure of one or more blood gasses in a whole blood sample of a mammal subject. Additionally or alternatively, the blood analyzer may be configured to measure concentrations of one or more electrolytes and/or metabolites in the blood sample and/or a hematocrit value of the blood sample and/or hemoglobin and hemoglobin-derivate values. For example, the blood analyzer may be configured to measure one or more of the following partial pressures or concentrations: *p*CO₂, *p*O₂, *p*H, Na⁺, K⁺, Ca²⁺, Cl⁻, Mg²⁺, glucose, lactate, creatinine, urea and hemoglobin and hemoglobin-derivate values.

In some embodiments, the blood analyzer may comprise a glass cuvette for accommodating a blood sample to be analyzed. The blood analyzer may further comprise an optical analysis unit. The optical analysis unit may comprise a light source for illuminating the blood sample accommodated in the glass cuvette and a detector, such as a spectrophotometer, for receiving light from illuminated blood sample accommodated in the class cuvette. During use, the glass cuvette deteriorates and the deterioration influences the resulting measurements, ultimately leading to a failure of the glass cuvette, also referred to as hemolyzer glass breakdown. Occurrence of such failure requires replacement of the glass cuvette or of a unit including the glass cuvette. Accordingly, in some embodiments, the hardware component is a glass cuvette of an optical analysis unit of the blood analyzer. To this end, deviations of measured MetHb levels, measured on one or more quality control samples from the associated target values for said quality control samples have been found to be a suitable performance parameter for failure prediction of a glass cuvette of a hemolyzer unit of a spectrophotometric detector for analyzing whole blood samples.

It will be appreciated, however, that embodiments of the method and system described herein may also be applied to other types of hardware components and/or other types of analyzer units. Examples of hardware components subject to maintenance include but are not limited to: sensor packs, valves, gaskets, membranes, light sources, etc. or other hardware components subject to gradual deterioration over time or subject to repeated use.

Generally, examples of an apparatus for analyzing biological samples may include an analyzer unit for use as point-of-care analyzers in healthcare facilities or analyzer units for use in laboratories. Examples of apparatus for analyzing biological samples may include apparatus for analyzing urine samples or other liquid biological samples, analyzers for analyzing tissue samples, etc.

The present disclosure relates to different aspects including the method described above, corresponding apparatus, systems, methods, and/or products, each yielding one or more of the benefits and advantages described in connection with one or more of the other aspects, and each having one or more embodiments corresponding to the embodiments described in connection with one or more of the other aspects and/or disclosed in the appended claims.

In particular, according to one aspect, disclosed herein are embodiments of a method for performing maintenance of an apparatus for analyzing biological samples, the apparatus including at least one hardware component subject to maintenance, the method comprising:
- causing a data processing system to perform the steps of the method according to the computer-implemented method described above and in the following;
- performing maintenance of the hardware component at a maintenance time no later than the estimated failure time.

According to another aspect, disclosed herein are embodiments of a data processing system configured to perform the steps of the computer-implemented method described above and in the following.

Here and in the following, the term data processing system is intended to comprise any suitably programmed computer or system of multiple computers, e.g. distributed data processing system, a virtual machine, a cloud-based computing system etc.

According to another aspect, disclosed herein are embodiments of a computer program product comprising executable program code configured to cause, when executed by a data processing system or processing unit, the data processing system or processing unit to perform the acts of the method described herein. The computer program product may be provided as a computer-readable medium, in particular a non-transitory medium, such as a CD-ROM, DVD, optical disc, memory card, flash memory, magnetic data storage de-vice, floppy disk, hard disk, etc. In other embodiments, a computer program product may be provided as a downloadable software package, e.g. on a web server for download over the internet or other computer or communication network.

The term processing unit is intended to comprise any circuit and/or device suitably adapted to perform some or all of the signal and/or data processing functions described herein. In particular, the term processing unit comprises a general- or special-purpose programmable microprocessor, such as a central processing unit (CPU) of a computer or of another data processing system, a digital signal processor (DSP), an application specific integrated circuits (ASIC), a programmable logic arrays (PLA), a field programmable gate array (FPGA), a special purpose electronic circuit, etc., or a combination thereof.

According to yet another aspect, disclosed herein are embodiments of a system comprising:
- at least one apparatus for analyzing biological samples, the apparatus including at least one hardware component subject to maintenance;
- a data processing system as described above and in the following, the data processing system being communicatively coupled to the at least on apparatus and configured to receive measurement results from the at least one apparatus.

### BRIEF DESCRIPTION OF THE DRAWINGS

Preferred embodiments of various aspects disclosed herein will be described in more detail in connection with the appended drawings, where:
FIG. 1 shows a schematic block diagram of an example of an apparatus for analyzing biological samples.
FIG. 2 schematically shows an example of a measurement unit of an apparatus for analyzing biological samples.
FIG. 3 shows a schematic block diagram of a system for analyzing biological samples.
FIG. 4 schematically illustrates an example of a computer-implemented method for supporting maintenance of an apparatus for analyzing biological samples.
FIG. 5 shows an example of a computed time series of a performance parameter.
FIG. 6 shows another example of a time series of a performance parameter.

### DETAILED DESCRIPTION

In the following, an embodiment of the method and other aspects disclosed herein will be described in the context of a glass cuvette of a blood analyzer. However, it will be appreciated that the method and other aspects disclosed herein may also be applied to other hardware components of a blood analyzer or of another type of apparatus for analyzing biological samples.

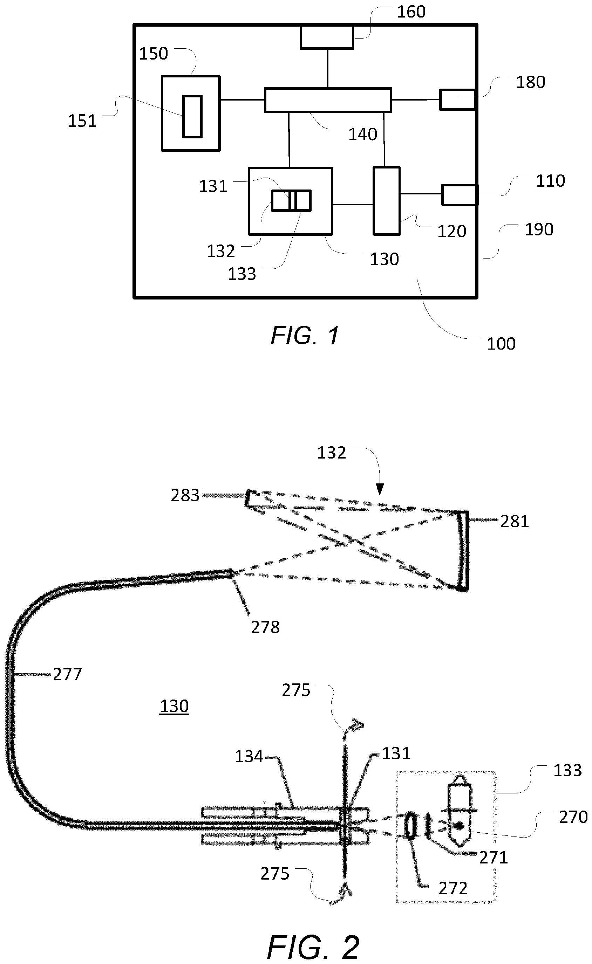

FIG. 1 shows a schematic block diagram of an apparatus for analyzing biological samples. In this example the apparatus is an analyzer unit for analyzing blood samples. The analyzer unit, generally designated by reference numeral 100, comprises an inlet 110 for receiving a blood sample, a fluid handling system 120, a measurement system 130 and a processing unit 140.

The measurement system 130 includes one or more sensors and/or detectors for obtaining respective measurement results. In particular, in the present example, the measurement system includes a spectrophotometric detector unit 132 for obtaining an absorption spectrum of the blood sample. To this end, the measurement system 130 comprises a glass cuvette 131 for accommodating a blood sample and an illumination unit 133 for illuminating the biological sample accommodated in the glass cuvette 131. When the analyzer unit 100 receives a blood sample at the inlet 110, the fluid handling system 120 feeds the received blood sample to the glass cuvette 131, and the measurement system 130 performs a photometric measurement to obtain an absorption spectrum. The analyzer unit 100 may then determine respective concentrations of one or more analytes in the blood sample, such as one or more of the following: levels of hemoglobin and/or hemoglobin-derivate values. Optical sensors/detectors suitable for measuring the above analytes are known as such in the art. An example of a spectrophotometric detector 132 will be described in more detail below with reference to FIG. 2. In some embodiments, the measurements system 130 may include alternative and/or additional sensors/detectors, such as one or more electrochemical sensors, e.g. as described in EP 2 147 307 B1 or in US 8,728,288 B2, and or another type of measurement chamber other than a glass cuvette.

After the measurement, the fluid handling system 120 may discard the blood sample from the glass cuvette 131 or other type of measurement chamber, e.g. into a suitable waste container (not explicitly shown). The measurement system 130 forwards the measurement results to the processing unit 140 for further processing. It will be appreciated that the measurement system 130 may forward raw measurement signals or pre-processed measurement signals or data, such as A/D converted signals, filtered signals, amplified signals and/or otherwise preprocessed signals or data to the processing unit 140.

Generally, it will be appreciated that the fluid handling system 120 may include one or more components, such as fluid conduits, gaskets, one or more pumps, valves and/or the like, all known as such in the art. Similarly, the measurement system 130 includes the one or more sensors/detectors and, optionally, other components, such as electrical circuitry, one or more excitation sources, one or more detectors, one or more electrodes, one or more reference electrodes, and/or the like, all known as such in the art. For example, an example of a blood analyzer unit is described in WO 18/104134.

The processing unit 140 may e.g. include a suitable programmed CPU and be configured for executing program code 151 to control operation of the analyzer unit and/or to process sensor/detector signals obtained by the measurement unit 130. To this end, the analyzer unit 100 may further comprise a data storage device 150, such as a hard drive, EEPROM, solid state drive, or the like. The data storage de-vice 150 may have stored thereon the program code 151 and/or other information. The processing unit 140 may thus load the program code 151 from the memory 150 and execute the loaded program code. In particular, the program code 151 may be configured to cause the processing unit 140 to compute a performance parameter or at least measurement results from which a performance parameter can be computed.

The analyzer unit 100 may comprise additional components, e.g. an output interface 160. The output interface may include a display, such as a touch screen, or another suitable output device for presenting measurement results and/or other relevant information to the user of the analyzer unit.

The analyzer unit 100 may include a communications interface 180 for wired or wireless data communication, e.g. via a local area network or other suitable computer network. The communications interface 180 may utilize Wi-Fi, Bluetooth or any other suitable wireless or wired communications technology, e.g. communications following one or more of the IEEE 802 suit of standards. To this end, the communications interface 180 may comprise a wired or wireless network adapter and/or other suitable circuitry or devices for providing wireless or wired data communications.

The analyzer unit 100 further comprises a housing 190 accommodating the inlet 110, the fluid transport system 120, the measurement system 130, the processing unit 140, the data storage device 150, the output interface 160 and the communications interface 180.

FIG. 2 schematically shows an example of a measurement system of an apparatus for analyzing biological samples. The measurement system 130 of FIG. 2 includes a sample illumination unit 133, a glass cuvette 131 and a spectrophotometric detector unit 132. A biological sample or a quality control sample may be fed into and out off the glass cuvette 131 by suitable conduits, as illustrated by arrows 275.

In the example of FIG. 2 the illumination unit 133 comprises a halogen lamp 270 or another suitable light source. The illumination unit 133 directs a light beam onto the glass cuvette 131, which comprises a blood sample or a quality-control (QC) sample, depending on whether the current measurement is a measurement of a biological sample or a QC measurement. The measurement system 130 further comprises a hemolyzer unit 134 configured to hemolyze the blood sample in the glass cuvette 131 by means of ultrasonic waves. Hemolyzing is a process, which ruptures the walls of the red blood cells in the sample, thereby causing the blood cells to release their content of hemoglobin. The light beam may be transmitted to the glass cuvette 131 through an infrared filter 271, and a biconvex lens 272. After passing through the glass cuvette 131, the light beam is directed towards the spectrophotometric detector unit 132, e.g. via an optical fiber 277 that directs the light through an aperture 278, e.g. a narrow slit, whereby the beam is directed towards a concave grating unit 281, which diffracts the light beam according to wavelength.

The concave grating unit 281 focuses light on a photodiode array 283, to which the diffracted light beam is transmitted. The photodiode array 283 may e.g. comprise a plurality, e.g. 128, photodiodes, and the array 283 may be arranged in such a manner that light comprising a range of wavelengths, e.g. of approximately 1.5 nm, in the diffracted light beam, strike a photodiode (not shown), which converts the light into a current substantially proportional to the light intensity which strikes it. By measuring the value of the current in each of the plurality of photodiodes of the photodiode array 283, a discrete intensity spectrum of the light beam after transmission through the sample is produced. From this intensity spectrum an absorption spectrum of the blood or QC sample comprised in the glass cuvette 131 may be determined by a processing unit of the apparatus, e.g. by processing unit 140 of the apparatus of FIG. 1. The absorption spectrum may e.g. be obtained in the wavelength range 478-672 nm or in another suitable wavelength range.

Generally, some embodiments of the apparatus for measuring biological samples may be controlled to perform quality control measurements, e.g. according to a predetermined schedule and/or based on certain trigger events, e.g. in response to a user input initiating a QC procedure, in response to an initialization of the apparatus, e.g. after a restart and/or responsive to other types of operational events. Quality control measurements may be performed on one or more predetermined quality control samples and serve to monitor the operational state of the apparatus and/or to calibrate certain aspects of the apparatus, e.g. to perform a calibration in respect of a possible wavelength shift of the spectrophotometric detector unit and/or other quantities.

In some embodiments a plurality of different types of quality control samples may be provided, e.g. quality control samples that include respective predetermined levels of one or more analytes. In one particular example, the apparatus may be provided with quality control samples including Sulforhodamine B in different concentrations. Increased reliability in the quality control of the spectrophotometric detector unit may thus be provided by measuring absorption spectra of QC samples at several analyte levels. By utilizing QC samples comprising a predetermined analyte, such as Sulforhodamine B, in different concentrations, it is ensured that the apparatus can measure blood parameters correctly over a wide range of component concentrations in blood samples.

An example of such quality control procedure is described in US6980285. It will be appreciated, however, that different embodiments may include different types of quality control samples, e.g. QC samples including other analytes, and/or employ different QC procedures based on these QC samples. The particular QC samples and procedures may generally be adapted to the specific type of sensor /detector employed by the apparatus.

As mentioned above, other examples of an apparatus for analyzing biological samples may include alternative or additional components, e.g. depending on the type of biological sample to be analyzed, the type of analyses to be performed, etc. For example, other embodiments of an apparatus for analyzing blood samples may include sensors/detectors other than a spectrophotometric detector. Yet further, other embodiments of an apparatus for analyzing biological samples may be configured to analyze biological samples other than blood samples, e.g. samples of other fluids, such as urine samples, or samples including tissue etc.

In any event, most apparatus for analyzing biological samples include one or more hardware components subject to maintenance, e.g. subject to manual adjustments, cleaning or even replacement of parts of the component or even the entire component. In the example of the spectrophotometric detector unit 132 described in connection with FIG. 2, it has turned out that the glass cuvette 131 is subject to maintenance, as the glass surface deteriorates due to the ultrasonic hemolyzing process, ultimately leading to glass breakdown of the cuvette.

An example of a process for estimating when such a glass breakdown will occur will be described in greater detail below. An accurate prediction of an estimated failure time of a hardware component allows maintenance of said component to be efficiently schedule, in particular in time to prevent the failure to occur though not unnecessarily early, thus preventing unscheduled down-time of the apparatus while reducing scheduled down-time and maintenance costs.

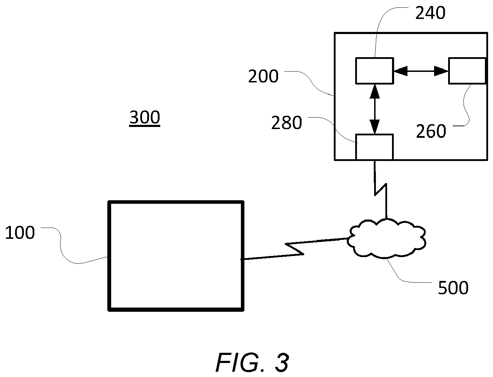

FIG. 3 shows a schematic block diagram of a system 300 for analyzing biological samples. The system 300 comprises an analyzer unit 100 for analyzing biological samples. The analyzer unit 100 may be an analyzer unit as described in connection with FIG. 1 or another suitable analyzer unit.

The system 300 further comprises a data processing system 200 communicatively coupled to the analyzer unit 100, e.g. via a suitable communications network 500, such as a local communications network of a healthcare facility and/or the internet and/or another suitable communications channel. In some embodiments, the system 300 may comprise a cloud based architecture, wherein cloud computing resources may be communicatively coupled to the analyzer unit 100 for processing data.

In FIG. 3, only a single analyzer unit 100 is shown. However, it will be appreciated that the data processing system 200 may be communicatively coupled to multiple analyzer units.

The data processing system 200 may be a single computer or a distributed data processing system comprising several computers, one or more virtual machines, and/or the like. The data processing system 200 comprises a communications interface 280 configured to communicate with the analyzer unit 100, e.g. via communications interface 180 of the analyzer unit of FIG. 1. The data processing system 200 further comprises a suitably programmed processing unit 240, e.g. including a CPU, and, optionally, a user interface 260, e.g. comprising a display and a keyboard, or a touchscreen or another suitable form of user interface. The data processing system 200 may configured to operate the user interface 260 as a maintenance interface that allows an authorized user of the data processing system to view and/or modify maintenance related information and/or other information pertaining to the analyzer unit 100. For example, the data processing system 200 may execute a device management program configured to provide device management functionality for a plurality of analyzer units 100. In particular, the data processing system 200 may be configured to compute and output an estimated failure time of a hardware component of the analyzer unit 100 based on measurement results received from the analyzer unit 100 as described herein.

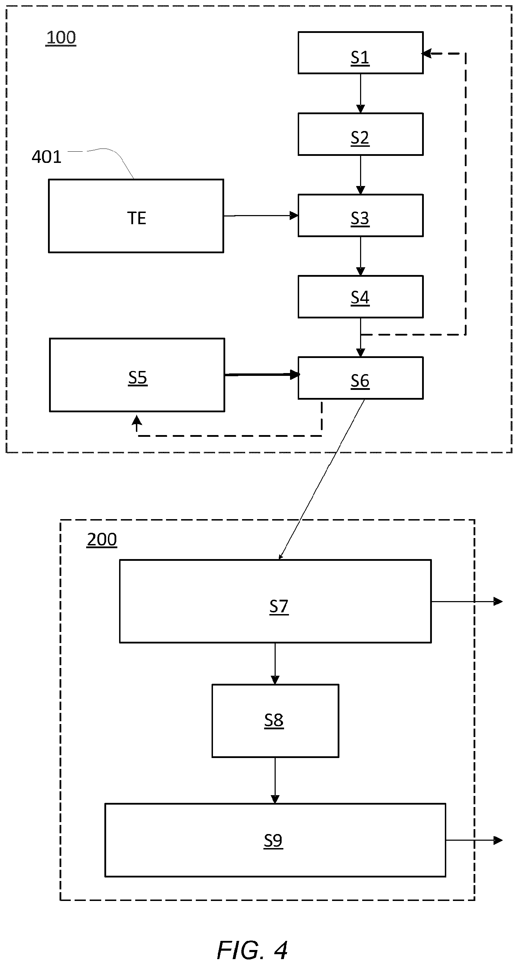

FIG. 4 schematically illustrates an example of a computer-implemented method for supporting maintenance of an apparatus for analyzing biological samples. Embodiments of the method may e.g. be performed by the system 300 of FIG. 3. The system 300 may include an analyzer unit 100 as described in connection with FIGs. 1 and 2.

In an initial step S1, the analyzer unit 100 performs a measurement on a blood sample, e.g. a sample of whole blood obtained from a human subject, such as from a patient at a healthcare facility. The measurement may be part of the normal operation of the apparatus at a healthcare facility and result in one or more measurement results for use during the diagnostics and/or therapy of a particular patient. The measurement may involve measuring an absorption spectrum of the blood sample after feeding the blood sample into a glass cuvette and after hemolyzing the blood sample, e.g. as described in connection with FIG. 2.

In step S2, the processing unit of the analyzer unit computes a measure of turbidity of the blood sample based on the absorption spectrum. The measure of turbidity is a measure of the cloudiness or haziness of the blood sample by individual particles (in particular suspended solids) that are generally invisible to the naked eye.

In step S3, based on the computed turbidity, the processing unit of the analyzer unit updates a correction index maintained by the processing unit. The correction index is thus an aggregated index computed based on a measure of turbidity derived from absorption spectra of multiple blood samples. For example, in one embodiment, the process computes a dot product of a vector-representation of the measured absorbance spectrum a coefficient vector derived by multivariate calibration that takes into account the different Hb spectra like HHb, O2Hb, COHb, MetHb, SHb and also bilirubin. The processing unit resets the correction index responsive to one or more trigger events 401, e.g. each time the analyzer unit is restarted and/or responsive to predetermined maintenance events.

In step S4, the processing unit of the analyzer unit performs one or more noise reducing and/or other data cleansing operations on the computed correction index, e.g. a low-pass filtering, moving average and/or other filter operations.

In step S5, the analyzer unit performs a quality control measurement. The quality control measurement includes obtaining an absorption spectrum of a quality control sample, i.e. a synthetic sample having a predetermined and known target concentration of one or more analytes. Such quality control measurements may be performed regularly, e.g. according to a predetermined quality control schedule and/or they may be triggered by one or more trigger events, e.g. after performing a predetermined number of measurements, after a restart of the system, etc.

In step S6, the processing unit computes a concentration of the one or more analytes of the quality control sample from the measured absorption spectrum of the quality control sample. The processing unit further applies, e.g. adds to, the correction index to the computed concentration and determines a bias, i.e. a deviation, between the computed concentration and the known target concentration of the quality control sample. It will be appreciated that the quality control process may involve quality control measurements on multiple different quality control samples, e.g. quality control samples including different levels of the analyte. Accordingly, some embodiments of the process may compute respective biases for the different types of quality control samples.

The analyzer unit 100 may forward the computed bias to a remote data processing system 200, e.g. to a cloud based system as described in connection with FIG. 3. It will be appreciated that, in other embodiments, the analyzer unit 100 may forward alternative or additional types of measurement results and, optionally other information, to the data processing system 200. For example, the analyzer unit 100 may forward the measured absorption spectra to the data processing system 200 so as to allow the data processing system 200 to perform the computation of the measurement result. Similarly, in other embodiments, the analyzer unit 100 may perform other types of corrections to the measurement results, e.g. based on other measures different from turbidity or no correction at all. Yet further, some embodiments of an analyzer unit forward measurement results that are derived from both quality control samples and from biological samples, e.g. measurements on quality control samples corrected based on measurements of biological samples as in the present example, or measurements of biological samples corrected based on quality control measurements. Other embodiments of an analyzer unit may forward measurement results based only on measurements of quality control samples or only based on blood or other biological samples.

The data processing system 200 receives the measurement results and computes an estimated failure time of a hardware component of the analyzer unit based on the received measurement results. The data processing system 200 may further receive additional data from the analyzer unit, e.g. information about performed maintenance operations, restarts of the analyzer unit, other operational data, and/or the like.

In the present example, the hardware component is the glass cuvette 131 of the hemolyzer unit 134 of the analyzer unit 100, and the data processing system 200 uses the computed bias associated with quality control measurements as a performance parameter to compute an estimated failure time of the glass cuvette 131. In particular, the computed bias is a bias corrected based on a correction index that is determined based on measured turbidity measures of blood samples that have been analyzed by the analyzer unit 100, e.g. as described in connection with steps S1 through S6 above. It will be appreciated that other embodiments of the process may compute estimated failure times of other hardware components of the analyzer unit and/or compute an estimated failure time based on another performance parameter, which may be based on other types of measurement results.

Generally, in initial step S7 the data processing system 200 performs one or more preprocessing steps on the received measured results from the analyzer unit, e.g. for computing a performance parameter different from the measurement results received from the analyzer unit and/or for filtering the performance parameter, etc. In the present example, the data processing system 200 uses the received measurement results, in particular the received bias, directly as a performance parameter and computes a time series of the performance parameter indicative of the value of the performance parameter over an observation period of time. The observation period may extend from an end time, e.g. current time at which the computation is performed or the time of the most recent measurement result, to a start time in the past.

The data processing system 200 may e.g. compute the time series as a moving median of the performance parameter from values received at different points in time.

The received values may thus represent the computed bias associated with multiple quality control measurements. The moving median may be computed over a suitable time window, e.g. a 7-day window or a time window of another suitable length.

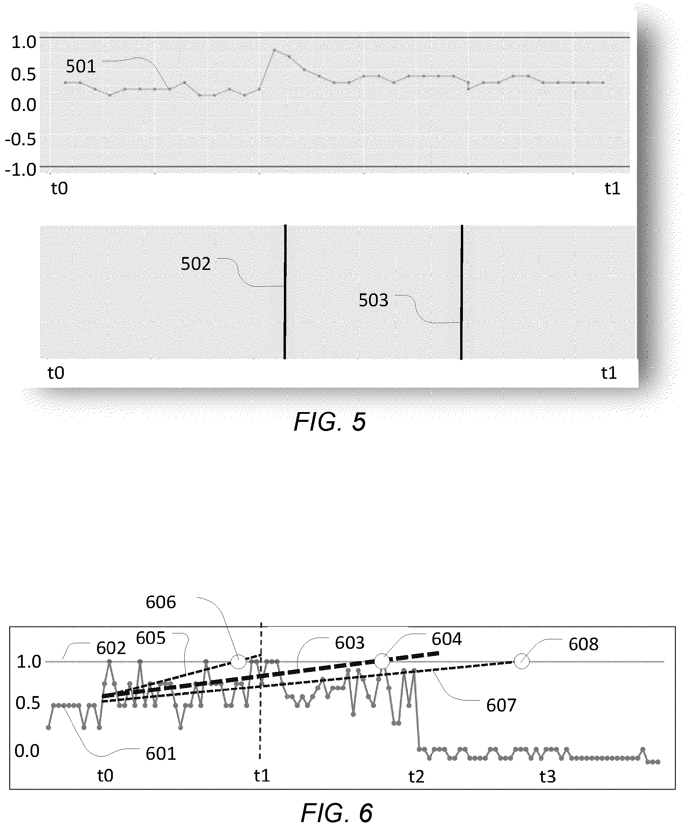

FIG. 5 shows an example of the thus computed time series of a performance parameter. In particular, FIG. 5 shows a time series 501 of a computed bias of quality control measurements corrected based on a correction index resulting from measurements of a turbidity measure on blood samples, as described above. In the example of FIG. 5, the time series is shown for an observation period of about 2 months. It will be appreciated that embodiments of the process may compute the time series over different observation periods, such as shorter or longer observation periods.

Generally, the observation period of time may have any suitable length, e.g. 1 week, several weeks, 1 month, several months or even longer. A suitable choice of observation period may depend on parameters such as the frequency of measurement results, the time scale of the deterioration process that leads to the component failure, etc. The observation period may be fixed or vary.

FIG. 5 further indicates times 502 and 503 at which certain maintenance events have occurred, e.g. the replacement of a sensor cassette and/or replacement of another part of the analyzer unit and/or another maintenance action that may have involved a restart of the analyzer unit. As can be seen from the time series 501, responsive to the maintenance events at times 502 and 503, the performance parameter may temporarily increase or decrease. Such temporary increase or decrease may e.g. be caused by the analyzer unit resetting one or more accumulated parameters that influence the computation of the measurement results in which the performance parameter is based, e.g. the correction factor discussed in connection with step S3 above. In order to avoid that such temporary changes of the performance parameter due to certain maintenance events influence the computation of an estimated failure time, some embodiments of the process may ignore values of the performance parameter obtained within a time window, such as a predetermined time window, after registration of a maintenance event and/or after registration of another operational event known to temporarily affect the performance parameter.

The data processing system 200 may further scale the performance parameter, e.g. compared to a predetermined maximum threshold value of the performance so as to arrive at a time series indicating a scaled performance parameter, e.g. expressed as a percentage of the performance parameter compared to the maximum threshold value.

FIG. 6 shows an example of a resulting time series 601 of a scaled performance parameter, in this example of the scaled bias described above. The scaled performance parameter is scaled relative to a predetermined maximum threshold value 602, in this example an upper limit of a range of acceptable values of the performance parameter.

In some embodiments, the data processing system 200 may output, e.g. display the current value of the, optionally scaled, performance parameter. The data processing system may further issue an alert or warning based on the current value of the performance parameter, e.g. responsive to the performance parameter exceeding a predetermined alert threshold, e.g. a predetermined percentage of the maximum threshold value.

Still referring to FIG. 4 and with continued reference to FIG. 6, in step S8, the data processing system 200 extrapolates the computed time series based on an observation window extending from a start time t0 in the past to an end time, which may be the current time t1 at which the computation is performed or a time of the most recent measurement result, which may be earlier than the current time. It will be appreciated that the length and position of the observation window may be different in different embodiments and depend on factors such as the amount of available data, the nature and/or time scales of the mechanisms resulting in the component failure to be predicted and/or the like. Generally, it will be appreciated that the current time may be a time at which the extrapolation is performed.

The extrapolation may extend to an extrapolation horizon at a future time t3, later than the end time of the observation period, in particular later than the current time. The extrapolation horizon may be different in different embodiments. Again the extrapolation horizon may depend on factors such as the amount of available data, the nature and/or time scales of the mechanisms resulting in the component failure to be predicted and/or the like. The observation window and/or the extrapolation horizon may be predetermined or be adaptively determined by the process.

In the embodiment of FIG. 6, the extrapolation is a linear extrapolation which has been found to be suitable for computing an estimated failure time. Moreover a linear extrapolation reduces the risk of overfitting noisy data, is computationally efficient and can be performed even based on relatively few data points. It will be appreciated that other embodiments may use other extrapolation models, e.g. non-linear models. The choice of extrapolation may depend on a priori knowledge of the mechanism leading to the component failure to be estimated. For example, if the process resulting in a component failure is known to accelerate, e.g. exponentially, a non-linear extrapolation, e.g. an exponential extrapolation, may be chosen.

In FIG. 6, a linear extrapolation 603 of the time series is shown. The linear interpolation was performed at time t1 (representing the current time of the extrapolation) and it extends beyond the current time t1 into the future. FIG. 6 also shows actual recorded observations obtained after the current time t1 of the extrapolation to allow a comparison of the time series 601 based on actual observations with the extrapolation 603.

Optionally, the process may further compute a confidence interval of the extrapolation, e.g. reflecting range of interpolations within which the extrapolation is expected to lie with a predetermined confidence, e.g. with 95% confidence. FIG. 6 shows the 95% confidence interval as extrapolation lines 605 and 607.

Still referring to FIG. 4 and with continued reference to FIG. 6, in S9, the process determines from the extrapolation when the performance parameter is expected to reach a critical level that would indicate an imminent risk of component failure. For example, a critical level may be defined as the extrapolated performance parameter extending outside a range of acceptable values. In the present example, the critical value is the maximum threshold value 602. Accordingly, the estimated failure time t2 is computed as the time 604 at which the interpolation 603 exceeds the maximum threshold value 602. It will be appreciated that other embodiments of the process may compute the estimated failure time from the extrapolation in a different manner. For example, some embodiments may determine an estimated future time at which the estimated value of the extrapolated time series fulfils a predetermined criterion indicative of a risk of component failure, e.g. exceeds a predetermined threshold, decreases below a predetermined threshold, or the like. The process may then determine the estimated failure time directly as the thus estimated future time or the process may otherwise compute the estimated failure time from the thus estimated future time. For example, some embodiments may subtract a predetermined margin from the thus estimated future time to arrive at the estimated failure time.

In any event, the process may output the calculated estimated failure time in a suitable manner, e.g. as part of a maintenance dashboard or other user interface allowing a maintenance operator to schedule maintenance of the analyzer unit. Optionally, the process may further compute a confidence interval for the estimated failure time, e.g. as respective intersections 606 and 608 of the extrapolations lines 605 and 607, respectively, representing the confidence interval of the extrapolation. Optionally, the data processing system may forward information about the estimated failure time to the analyzer unit 100 and/or to another system. For example, the data processing system may 200 forward the estimated failure time to a maintenance scheduling system and/or an inventory management system allowing scheduling of a maintenance visit of an operator and/or ordering of spare parts. Accordingly, based on the estimated failure time, the operator of the analyzer may take maintenance decisions as to when to perform maintenance of the hardware component so as to prevent actual component failure while avoiding unnecessarily frequent maintenance.

In some embodiments, the estimated failure time may be displayed only when the estimated failure time is sooner than a predetermined margin, e.g. within a month, within 6 months or within a year from the current time. Alternatively or additionally, the process may issue an alert or warning when the estimated failure time is sooner than a predetermined margin. Yet further, the computation of the estimated failure time may be performed subject to one or more trigger conditions being fulfilled. For example, in some embodiments the extrapolation is performed only when a minimum number of data points are available in the time series and/or only when the computed time series covers a sufficiently long observation period and/or when the performance parameter lies outside a nominal range, e.g. for at least a predetermined period of time.

It will be appreciated that the steps of the process of FIG. 4 may be distributed between the analyzer unit 100 and the data processing system 200 in a different manner. For example, in one embodiment, all steps except for the actual measurement steps may be performed by a remote data processing system 200. In another embodiment, the entire process may be performed locally by the analyzer unit 100, i.e. without the need for a remote data processing system 200.

Generally, all terms used herein are to be interpreted according to their ordinary meaning in the relevant technical field, unless a different meaning is clearly given and/or is implied from the context in which it is used.

Reference has been made herein to various embodiments. However, a person skilled in the art would recognize numerous variations to the described embodiments that would still fall within the scope of the claims.

For example, the method embodiments described herein disclose example methods through steps being performed in a certain order. However, it is recognized that these sequences of events may take place in another order without departing from the scope of the claims. Furthermore, some method steps may be performed in parallel even though they have been described as being performed in sequence. Thus, the steps of any methods disclosed herein do not have to be performed in the exact order disclosed, unless a step is explicitly described as following or preceding another step and/or where it is implicit that a step must follow or precede another step.

In the same manner, it should be noted that in the description of embodiments, the partition of functional blocks into particular units is by no means intended as limiting. Contrarily, these partitions are merely examples. Functional blocks described herein as one unit may be split into two or more units. Furthermore, functional blocks described herein as being implemented as two or more units may be merged into fewer (e.g. a single) unit.

Any feature of any of the embodiments disclosed herein may be applied to any other embodiment, wherever suitable. Likewise, any advantage of any of the embodiments may apply to any other embodiments, and vice versa.

Hence, it should be understood that the details of the described embodiments are merely examples brought forward for illustrative purposes, and that all variations that fall within the scope of the claims are intended to be embraced therein.

## Claims

1. A computer-implemented method for supporting maintenance of an apparatus for analyzing biological samples, the apparatus including at least one hardware component subject to maintenance, the method comprising:
- receiving a plurality of measurement results, each measurement result being derived from a measurement performed by the apparatus for analyzing biological samples on a biological sample or on a quality control sample, the plurality of measurement results including measurement results obtained at different times;
- computing a time series of a performance parameter from the plurality of measurement results, the performance parameter being indicative of an operational state of said at least one hardware component, the time series representing a period of time having a start time and an end time, the end time being no later than a current time;
- extrapolating, using a predetermined extrapolation model, the computed time series to obtain an extrapolated time series of the performance parameter, the extrapolated time series including one or more estimated values of the performance parameter at one or more future times later than the end time, in particular later than the current time;
- comparing the one or more estimated values of the performance parameter with a predetermined condition indicative of a risk of component failure of said component to determine an estimated future time at which said predetermined condition is fulfilled;
- determining an estimated failure time from the estimated future time.

2. A method according to claim 1, further comprising outputting the estimated failure time.

3. A method according to any one of the preceding claims, wherein the performance parameter is indicative of a deviation of a measured value from a target value associated with a quality control sample, the measured value resulting from a measurement of a quantity performed by the apparatus on said quality control sample.

4. A method according to claim 3, wherein the measured value is a corrected measured value resulting from a measurement of a quantity performed by the apparatus on said quality control sample and corrected based on one or more previous measurements performed on respective biological samples.

5. A method according to any one of the preceding claims, further comprising determining a confidence interval associated with the determined estimated failure time.

6. A method according to any one of the preceding claims, wherein extrapolating comprises performing a linear extrapolation.

7. A method according to any one of the preceding claims, wherein computing the time series comprises one or more data cleansing operations.

8. A method according to any one of the preceding claims, wherein computing the time series comprises:
- detecting one or more operational events, and
- discarding one or more of the received measurement results, the one or more discarded measurement results resulting from measurements performed at a time subsequent to the detected operational event.

9. A method according to claim 8, wherein the operational event includes a restart of the apparatus and/or one or more predetermined maintenance activities.

10. A method according to claim 8 or 9, wherein the one or more discarded measurement results result from measurements performed during a predetermined period after the detected event.

11. A method according to any one of claims 7 through 10, wherein computing the time series comprises computing a moving median and/or a low-pass filtering of the time series.

12. A method according to any one of the preceding claims, wherein receiving the measurement results comprises receiving the measurement results from the apparatus via a communications network and performing some or all of the steps of computing, extrapolating, comparing and determining by a data processing system remote from the apparatus.

13. A method according to any one of the preceding claims, wherein the apparatus is a blood analyzer for analyzing blood samples.

14. A method according to claim 13, wherein the hardware component is a glass cuvette of an optical analysis unit of the blood analyzer.

15. A method according to any one of the preceding claims; wherein comparing comprises comparing the one or more estimated values of the performance parameter with a predetermined target range of the performance parameter to determine an estimated future time at which one or more of the estimated values lie outside the predetermined target range.

16. A method for performing maintenance of an apparatus for analyzing biological samples, the apparatus including at least one hardware component subject to maintenance, the method comprising:
- causing a data processing system to perform the steps of the method according to any one of claims 1 through 15;
- performing maintenance of the hardware component at a maintenance time no later than the estimated failure time.

17. A data processing system configured to perform the steps of the method according to any one of claims 1 through 15.

18. A computer program product comprising program code configured to cause, when executed by a data processing system, the data processing system to perform the steps of the method according to any one of claims 1 through 15.

19. A system comprising:
- at least one apparatus for analyzing biological samples, the apparatus including at least one hardware component subject to maintenance;
- a data processing system according to claim 17, the data processing system being communicatively coupled to the at least one apparatus and configured to receive measurement results from the at least one apparatus.
